# FASCICULE DE BREVET EUROPEEN

(11) **EP 2 241 554 B1**
(45) Date de publication et mention de la délivrance du brevet: **07.09.2011**
(21) Numéro de dépôt: 10290166.7
(22) Date de dépôt: 30.03.2010
(51) Int. Cl.: C07D 223/16

(54) **Nouveau procédé de synthèse de l'ivabradine et de ses sels d'addition à un acide pharmaceutiquement acceptable**
Verfahren zur Herstellung von Ivabradin und dessen pharmazeutisch verträglichen Säureadditionssalzen
Process for the synthesis of ivabradine and its pharmaceutically acceptable acid addition salts

(30) Priorité: 31.03.2009 FR 0901555
(43) Date de publication de la demande: 20.10.2010
(73) Titulaire: Les Laboratoires Servier, 92284 Suresnes Cedex (FR)
(72) Inventeur: Peglion, Jean-Louis, 78110 Le Vesinet (FR); Dessinges, Aimée, 92500 Rueil Malmaison (FR); Serkiz, Bernard, 77170 Servon Brie Comte Robert (FR)

(56) Documents cités:
- EP-A- 0 534 859
- WO-A-2005/110993
- GB-A- 1 140 049
- ROUSSELET ET AL: "Copper(I)-induced addition of amines to unactivated nitriles: The first general one-step synthesis of alkyl amidines" TETRAHEDRON LETTERS, ELSEVIER, AMSTERDAM, vol. 34, no. 40, 1 octobre 1993 (1993-10-01), pages 6395-6398, XP022406486 ISSN: 0040-4039

## Description

La présente invention concerne un procédé de synthèse de l'ivabradine de formule (**I**) : 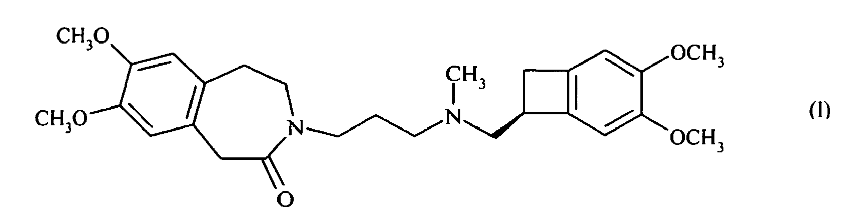 ou 3-{3-[{[(7*S*)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrites dans le brevet européen EP 0 534 859.

Ce brevet décrit la synthèse du chlorhydrate de l'ivabradine à partir du composé de formule (II) : 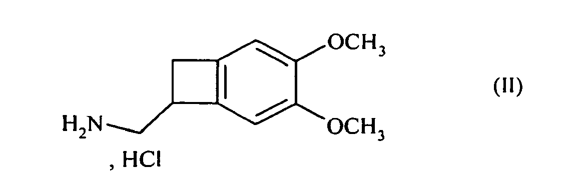 qui est transformé en composé de formule (III) : 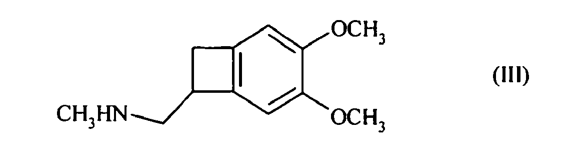 qui est dédoublé pour conduire au composé de formule (IV) : 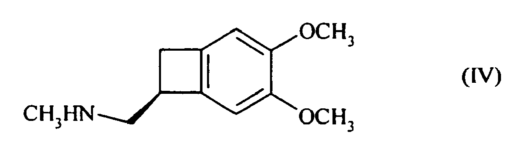 qui est mis en réaction avec le composé de formule (V) : 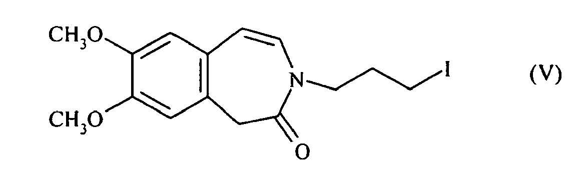 pour conduire au composé de formule (VI) : 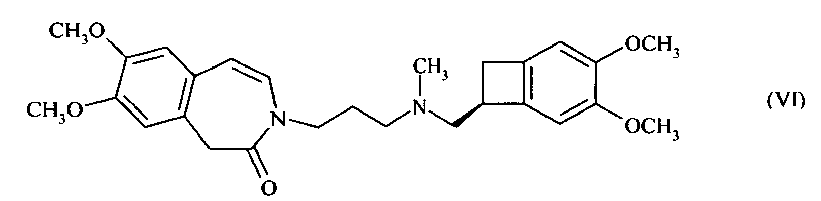 dont l'hydrogénation catalytique conduit à l'ivabradine, qui est alors transformée en son chlorhydrate.

L'inconvénient de cette voie de synthèse est de ne conduire à l'ivabradine qu'avec un rendement de l'ordre de 0,6%.

Compte-tenu de l'intérêt pharmaceutique de ce composé, il était important de pouvoir y accéder avec un procédé de synthèse performant, conduisant à l'ivabradine avec un bon rendement.

La présente invention concerne un procédé de synthèse du composé de formule (VII), sous forme racémique ou optiquement active : 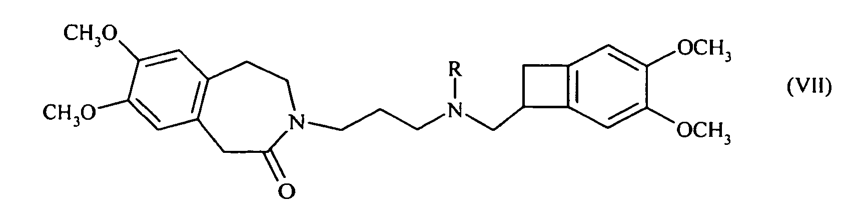 dans laquelle R représente un atome d'hydrogène ou un groupement méthyle, caractérisé en ce que le composé de formule (VIII) : 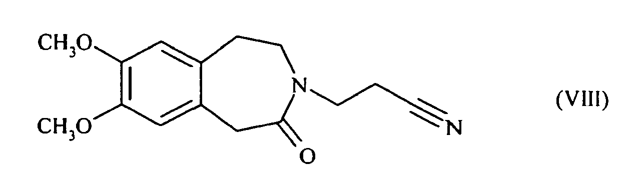 est mis en réaction avec le composé de formule (IX), sous forme racémique ou optiquement active, sous forme de base libre ou de sel : 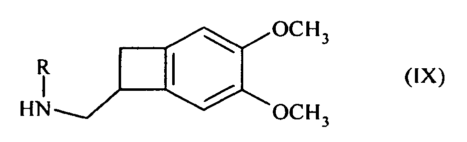 dans laquelle R est tel que défini précédemment,
en présence d'un sel de métal de transition ou de lanthanide,
dans un solvant,
pour conduire au composé de formule (X), sous forme racémique ou optiquement active : 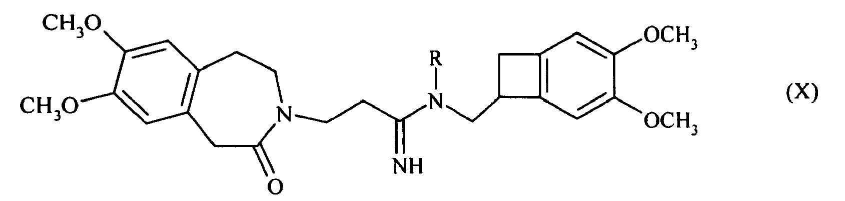 qui est transformé en composé de formule (VII) par action d'un agent donneur d'hydrure.

Dans un mode de réalisation préféré de l'invention, le composé de formule (IX) est sous forme optiquement active, et plus particulièrement de configuration (*S*).

Dans le cas où R représente un atome d'hydrogène, le produit de la réaction du composé de formule (X) avec l'agent donneur d'hydrure est alors le composé de formule (XI), cas particulier des composés de formule (VII) : 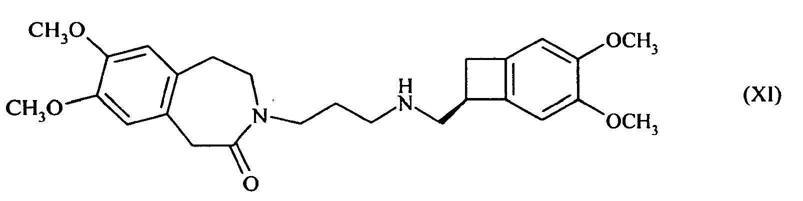 qui peut être *N*-méthylé pour conduire à l'ivabradine de formule (I) : 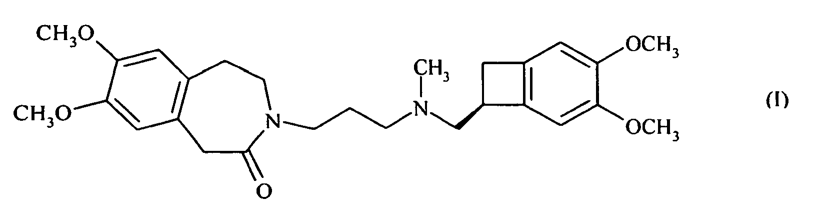 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Dans le cas ou R représente un groupement méthyle, le produit de la réaction du composé de formule (X) avec l'agent donneur d'hydrure est alors l'ivabradine de formule (I), cas particulier des composés de formule (VII) : 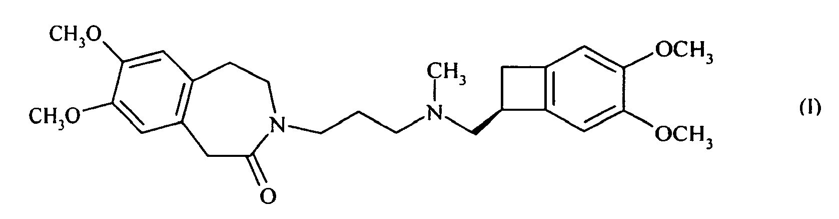 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Dans un autre mode de réalisation préféré de l'invention, le composé de formule (IX) est sous forme racémique.

Dans le cas où R représente un atome d'hydrogène, le produit de la réaction du composé de formule (X) avec l'agent donneur d'hydrure est alors le composé racémique de formule (XII), cas particulier des composés de formule (VII) : 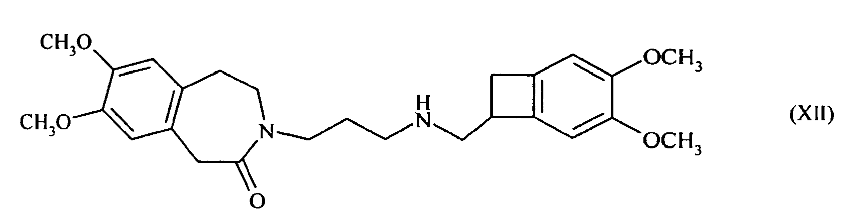 qui peut être *N*-méthylé pour conduire au composé racémique de formule (XIII) : 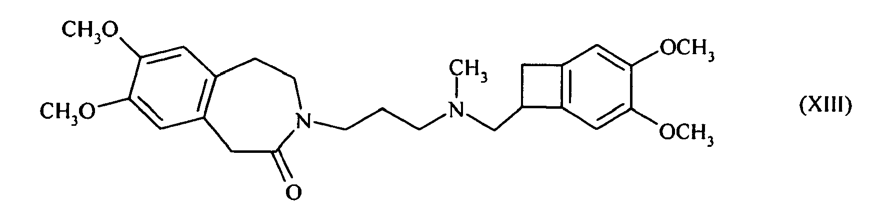 dont la résolution optique conduit à l'ivabradine de formule (I) : 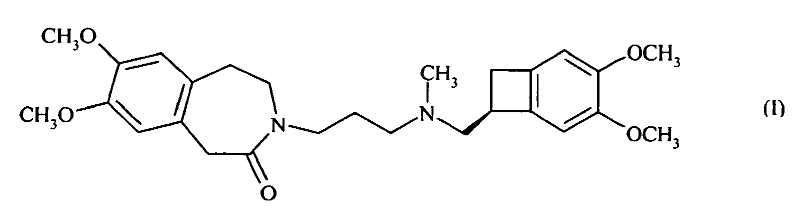 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Dans le cas où R représente un groupement méthyle, le produit de la réaction du composé de formule (X) avec l'agent donneur d'hydrure est alors le composé racémique de formule (XIII), cas particulier des composés de formule (VII) : 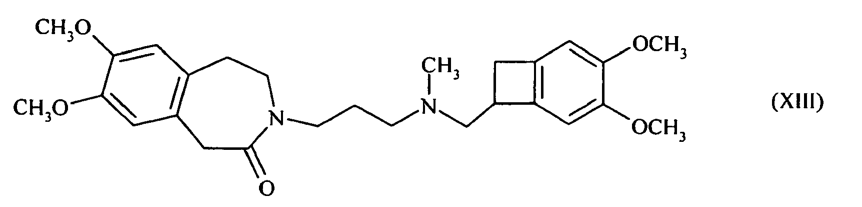 dont la résolution optique conduit à l'ivabradine de formule (I) : 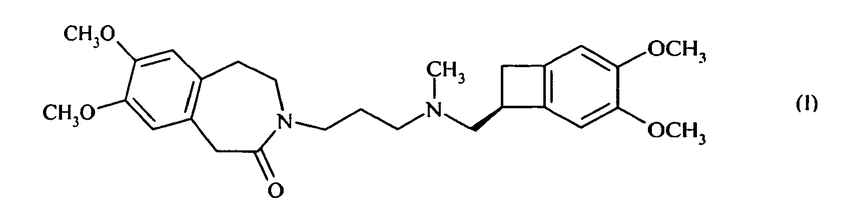 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Parmi les sels de métaux de transition ou de lanthanides pouvant être utilisés pour effectuer la réaction entre le composé de formule (VIII) et le composé de formule (IX), on peut citer à titre non limitatif le chlorure de cuivre(I), le bromure de cuivre(I), l'iodure de cuivre(I), le trifluorométhanesulfonate d'yttrium(III), le trifluorométhanesulfonate de lanthane(III), le trifluorométhanesulfonate de praséodyme(III), le trifluorométhanesulfonate de néodyme(III), le trifluorométhanesulfonate de samarium(III), le trifluorométhanesulfonate d'europium(III), le trifluorométhanesulfonate de gadolinium(III), le trifluorométhanesulfonate de terbium(III), le trifluorométhanesulfonate de dysprosium(III), le trifluorométhanesulfonate d'holmium(III), le trifluorométhanesulfonate d'erbium(III) et le trifluorométhanesulfonate de lutécium(III).

Le sel de métal de transition préférentiellement utilisé pour effectuer la réaction entre le composé de formule (VIII) et le composé de formule (IX) est le chlorure de cuivre(I).

Parmi les solvants pouvant être utilisés pour effectuer la réaction entre le composé de formule (VIII) et le composé de formule (IX), on peut citer à titre non limitatif :
- les solvants alcooliques, notamment le méthanol, l'éthanol et l'isopropanol ;
- le diméthylsulfoxyde (DMSO) ;
- le *N*,*N*-diméthylformamide (DMF) ;
- la *N*-méthylpyrrolidone (NMP).

Le solvant préférentiellement utilisé pour effectuer la réaction entre le composé de formule (VIII) et le composé de formule (IX) est le méthanol.

Parmi les agents donneurs d'hydrure pouvant être utilisés pour effectuer la transformation du composé de formule (X) en composé de formule (VII), on peut citer à titre non limitatif le tétraborohydrure de sodium, le cyanoborohydrure de sodium, ainsi que les complexes borane-morpholine et borane-diméthylamine.

Parmi les solvants pouvant être utilisés pour effectuer la transformation du composé de formule (X) en composé de formule (VII), on peut citer à titre non limitatif :
- les solvants alcooliques, notamment le méthanol, l'éthanol et l'isopropanol ;
- le *N*,*N*-diméthylformamide (DMF) ;
- la *N*-méthylpyrrolidone (NMP).

Les composés de formules (VIII) et (X), sont des produits nouveaux, utiles comme intermédiaires de synthèse dans l'industrie chimique ou pharmaceutique, notamment dans la synthèse de l'ivabradine, de ses sels d'addition à un acide pharmaceutiquement acceptable et de leurs hydrates, et font à ce titre partie intégrante de la présente invention.

Les exemples ci-dessous illustrent l'invention.

### Liste des abréviations utilisées :

DMF : *N*,*N*-diméthylformamide
IR : infrarouge

Les points de fusion (PF) ont été mesurés au Micro Köfler.(MK)

### EXEMPLE 1 : 3-(7,8-Diméthoxy-2-oxo-1,2,4,5-tétrahydro-3H-3-benzazépin-3-yl) propanenitrile

On solubilise 2 g (9 mmoles) 7,8-diméthoxy-1,3,4,5-tétrahydro-2*H*-3-benzazépin-2-one dans 30 mL de DMF. On ajoute à cette solution, à 25°C, 432 mg (10,8 mmoles, 1,2 équivalent) d'hydrure de sodium en suspension à 60% dans l'huile. On laisse agiter 30 minutes à température ambiante puis on ajoute une solution de 0,9 mL (10,8 mmoles, 1,2 équivalent) de 3-bromopropionitrile dans 10 mL de DMF. On chauffe ensuite 24 heures à 50°C puis on évapore le solvant. Le résidu est repris au dichlorométhane, lavé à l'eau, séché sur MgSO₄, filtré et évaporé à sec. On obtient 4,1 g d'un résidu qui est purifié par flash-chromatographie sur 300g de silice (éluant = dichlorométhane/éthanol :95/5).On obtient 630 mg de produit du titre sous forme d'une huile et on récupère 1 g de 7,8-diméthoxy-1,3,4,5-tetrahydro-2*H*-3-benzazépin-2-one n'ayant pas réagit (solide blanc, PF=196-198°C).
Rendement = 26%
IR (pur) : v= 2247, 1648, 1609, 1518, 1246, 1220, 1104 cm⁻¹.

### EXEMPLE 2 : 3-[3-({[(7S)-3,4-diméthoxybicyclo[4.2.0]octa-1,3,5-trién-7-yl]méthyl}amino)propyl]-7,8-diméthoxy-1,3,4,5-tétrahydro-2H-3-benzazépin-2-one

### Stade 1 : N-{[(7S)-3,4-diméthoxybicyclo[4.2.0]octa-1,3,5-trién-7-yl]méthyl}-3-(7,8-diméthoxy-2-oxo-1,2,4,5-tétrahydro-3H-3-benzazépin-3-yl)propanimidamide

Sous azote, on solubilise 630 mg (3,27 mmoles, 1,5 équivalent) de chlorhydrate de 1-[(7*S*)-3,4-diméthoxybicyclo [4.2.0]octa-1,3,5-trién-7-yl]méthanamine dans 10 mL de méthanol. On ajoute à cette solution 0,46 mL(3,27 mmoles, 1,5 équivalent) de triéthylamine et 260 mg (2,62 mmoles) de chlorure de cuivre(I) (pureté : 90%). On additionne ensuite goutte à goutte 600 mg (2,18 mmoles) de 3-(7,8-diméthoxy-2-oxo-1,2,4,5-tétrahydro-3*H*-3-benzazépin-3-yl)propanenitrile en solution dans 10 mL de méthanol. On chauffe à reflux pendant 12 heures puis on refroidit à température ambiante et on ajoute 5 mL de solution aqueuse de soude à 35% et 30 mL de dichlorométhane. La phase organique est extraite, séchée sur MgSO₄, filtrée puis évaporée à sec. On obtient 1,08 g d'une huile marron contenant 47% de produit attendu. Cette huile est engagée sans purification dans l'étape suivante.

### Stade 2 : 3-[3-({[(7S)-3,4-diméthoxybicyclo[4.2.0]octa-1,3,5-trién-7-yl]méthyl}amino) propyl]-7,8-diméthoxy-1,3,4,5-tétrahydro-2H-3-benzazépin-2-one

On solubilise 1 g de produit obtenu au stade 1 (contenant 47% d'amidine) dans 15 mL de méthanol puis on refroidit cette solution à 0°C et on ajoute 100 mg (2,61 mmoles, 1,2 équivalent) de tétraborohydrure de sodium. On laisse agiter une nuit à température ambiante puis on ajoute 5,3 mL de solution aqueuse de soude à 20% et 20 mL de dichlorométhane. On agite vigoureusement 15 minutes. La phase organique est ensuite extraite, lavée à l'eau, séchée sur MgSO₄, filtrée puis évaporée à sec. On obtient 1 g d'une huile qui est purifiée par flash-chromatographie sur 100g de silice (éluant = dichlorométhane/éthanol/NH₄OH: 90/10/1) pour conduire à 300 mg de produit attendu sous forme d'une huile.
Rendement = 30% (sur 2 étapes)
IR (pur) : ν = 3302, 1649 cm⁻¹ .

### EXEMPLE 3 : 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

### Stade 1 : chlorhydrate de 3-[3-({[(7S)-3,4-diméthoxybicyclo[4.2.0]octa-1,3,5-trién-7-yl] méthyl}amino)propyl]-7,8-diméthoxy-1,3,4,5-tétrahydro-2H-3-benzazépin-2-one

On solubilise 300mg (0,65 mmole) de l'amine obtenue à l'exemple 2, stade 2 dans 10 mL d'acétonitrile. On ajoute à cette solution 0,65 mL (1,3 mmole, 2 équivalents) d' une solution acide chlorhydrique 2M dans l'éther diéthylique. On agite 15 minutes à 25°C puis on évapore à sec. Le produit est cristallisé dans 20 mL d'acétone. Le solide est filtré et séché. On obtient 230 mg de cristaux blancs.
Rendement =71%
PF = 204-206°C

### Stade 2 : 3-{3-[{[(7S)-3,4-diméthoxybicyclo[4.2.0]octa-1,3,5-trién-7-yl]méthyl} (méthyl)amino]propyl}-7,8-diméthoxy-1,3,4,5-tétrahydro-2H-3-benzazépin-2-one

On solubilise 180 mg (0,36 mmole) de chlorhydrate obtenu au stade 1 dans un mélange de 10 mL de méthanol et 5 mL de dichlorométhane. On ajoute à cette solution 0,04 mL (0,54 mmole, 1,5 équivalent) de formaldéhyde (37% dans l'eau) et un grain de vert de bromocrésol. On ajoute une solution aqueuse d'acide chlorhydrique 1N jusqu'à pH=4 (couleur jaune de la solution) puis on laisse agiter 30 minutes à 25°C. On ajoute ensuite 23 mg (0,36 mmole) de cyanoborohydrure de sodium, on laisse agiter 12h à 25°C en maintenant le pH à 4, puis on évapore à sec. On obtient 250 mg d'une huile qui est purifiée par flash-chromatographie sur 100 g de silice (éluant = dichlorométhane/éthanol/NH₄OH : 90/10/1) pour conduire à 100 mg de produit du titre sous forme d'une huile incolore qui cristallise à température ambiante.
Rendement = 58%
PF = 98-100°C

### EXEMPLE 4 : Chlorhydrate du 3-{3-[{[(7S)-3,4-diméthoxybicyclo[4.2.0]octa-1,3,5-trién-7-yl]méthyl}(métbyl)amino]propyl}-7,8-diméthoxy-1,3,4,5-tétrahydro-2H-3-benzazépin-2-one

Le chlorhydrate du produit obtenu à l'exemple 3, stade 2 est préparé en suivant le mode opératoire décrit dans le brevet EP 0 534 859 (Exemple 2, stade E).

### EXEMPLE 5 : 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

### Stade 1 : N-{[(7S)-3,4-diméthoxybicyclo[4.2.0]octa-1,3,5-trién-7-yl]méthyl}-3-(7,8-diméthoxy-2-oxo-1,2,4,5-tétrahydro-3H-3-benzazépin-3-yl)-N-méthylpropanimidamide

Sous azote, on solubilise 691 mg (2,83 mmoles, 1,5 équivalent) de chlorhydrate de 1-[(7*S*)-3,4-diméthoxybicyclo[4.2.0]octa-1,3,5-trién-7-yl]-*N*-méthylméthanamine dans 10 mL de méthanol. On ajoute à cette solution 0,4 ml (2,83 mmoles, 1,5 équivalent) de triéthylamine et 224 mg (2,26 mmoles) de chlorure de cuivre(I) (pureté : 90%). On additionne ensuite goutte à goutte 520 mg (1,89 mmoles) de 3-(7,8-diméthoxy-2-oxo-1,2,4,5-tétrahydro-3*H-*3-benzazépin-3-yl)propanenitrile en solution dans 10 mL de méthanol. On chauffe à reflux pendant 24 heures puis on refroidit à température ambiante et on ajoute 5 mL de solution aqueuse de soude à 35% et 30 mL de dichlorométhane. La phase organique est extraite, séchée sur MgSO₄, filtrée puis évaporée à sec. On obtient 1,08 g d'une huile marron contenant 46% de produit attendu. Cette huile est engagée sans purification dans l'étape suivante.

### Stade2 : 3-{3-[{[(7S)-3,4-diméthoxybicyclo [4.2.0]octa-1,3,5-trién-7-yl]méthyl} (méthyl)amino]propyl}-7,8-diméthoxy-1,3,4,5-tétrahydro-2H-3-benzazépin-2-one

On solubilise 1 g de produit obtenu au stade 1 (contenant 46% d'amidine) dans 15 mL de méthanol puis on ajoute à température ambiante 86 mg (2,26 mmoles, 1,2 équivalent) de tétraborohydrure de sodium. On laisse agiter une nuit à température ambiante puis on ajoute 5 mL de solution aqueuse de soude à 20% et 20 mL de dichlorométhane. On agite vigoureusement 15 minutes. La phase organique est ensuite extraite, lavée à l'eau, séchée sur MgSO₄, filtrée puis évaporée à sec. On obtient 1 g d'une huile qui est purifiée par flash-chromatographie sur 100g de silice (éluant = dichlorométhane/éthanol/NH₄OH : 90/10/1) pour conduire à 210 mg de produit du titre sous forme d'une huile.
Rendement = 24% (sur 2 étapes)
IR (pur) : ν = 1633, 831-672 cm⁻¹

### EXEMPLE 6 : Chlorhydrate du 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

Le chlorhydrate du produit obtenu à l'exemple 5, stade 2 est préparé en suivant le mode opératoire décrit dans le brevet EP 0 534 859 (Exemple 2, stade E).

### EXEMPLE 7 : Chlorhydrate du 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

A partir de la (3,4-diméthoxybicyclo [4.2.0]octa-1,3,5-trién-7-yl)méthanamine racémique et en suivant le protocole décrit par l'enchaînement des exemples 2 et 3, on obtient la 3-{3-[[(3,4-diméthoxybicyclo[4.2.0]octa-1,3,5-trién-7-yl)méthyl](méthyl)amino]propyl}-7,8-diméthoxy-1,3,4,5-tétrahydro-2*H*-3-benzazépin-2-one.

2,1 g de ce composé racémique sont ensuite séparés sur une colonne de 60 cm x 60 mm packée avec 2,1 kg de phase Chiralpak ® AD (granulométrie 20 µm). L'éluant utilisé est un mélange éthanol / acétonitrile / diéthylamine (10/ 90 /0,1 en volume) à un débit de 50 mL / min. Le détecteur ultra-violet associé est utilisé à un longueur d'onde de 280 nm. On obtient 0,95g de l'énantiomère de configuration (R) sous forme de meringue blanche puis 0,95g de l'énantiomère de configuration (*S*) également sous forme de meringue blanche.

Le chlorhydrate de l'énantiomère de configuration (*S*) est ensuite obtenu en suivant le mode opératoire décrit dans le brevet EP 0 534 859 (Exemple 2, stade E).

### EXEMPLE 8 : Chlorhydrate du 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

A partir de la (3,4-diméthoxybicyclo[4.2.0]octa-1,3,5-trién-7-yl)-*N*-méthylméthanamine racémique et en suivant le protocole décrit à l'exemple 5, on obtient la 3-{3-[[(3,4-diméthoxybicyclo[4.2.0]octa-1,3,5-trién-7-yl)méthyl](méthyl)amino]propyl}-7,8-diméthoxy-1,3,4,5-tétrahydro-2*H*-3-benzazépin-2-one.

2,1 g de ce composé racémique sont ensuite séparés sur une colonne de 60 cm x 60 mm packée avec 2,1 kg de phase Chiralpak ® AD (granulométrie 20 µm). L'éluant utilisé est un mélange éthanol / acétonitrile / diéthylamine (10/90/0,1 en volume) à un débit de 50 mL / min. Le détecteur ultra-violet associé est utilisé à un longueur d'onde de 280 nm. On obtient 0,95g de l'énantiomère de configuration (R) sous forme de meringue blanche puis 0,95g de l'énantiomère de configuration (*S*) également sous forme de meringue blanche.

Le chlorhydrate de l'énantiomère de configuration (*S*) est ensuite obtenu en suivant le mode opératoire décrit dans le brevet EP 0 534 859 (Exemple 2, stade E).

## Revendications

1. Procédé de synthèse du composé de formule (VII), sous forme racémique ou optiquement active : 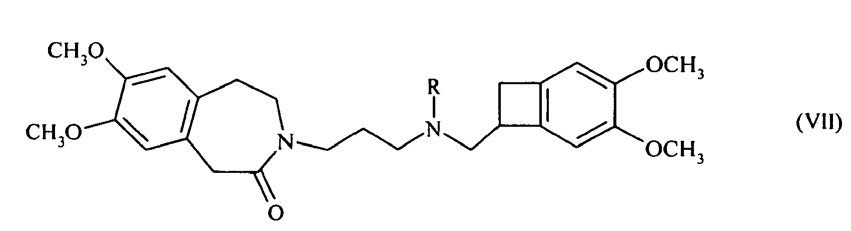 dans laquelle R représente un atome d'hydrogène ou un groupement méthyle,
**caractérisé en ce que** le composé de formule (VIII) : 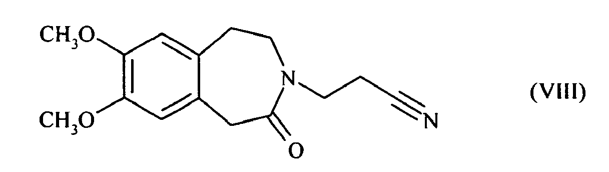 est mis en réaction avec le composé de formule (IX), sous forme racémique ou optiquement active, sous forme de base libre ou de sel : 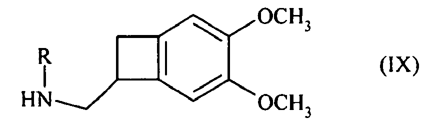 dans laquelle R est tel que défini précédemment,
en présence d'un sel de métal de transition ou de lanthanide,
dans un solvant,
pour conduire au composé de formule (X), sous forme racémique ou optiquement active : 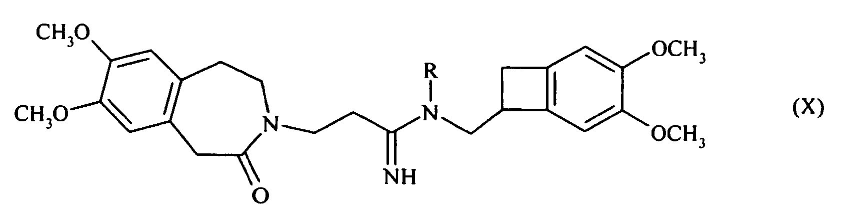 qui est transformé en composé de formule (VII) par action d'un agent donneur d'hydrure.

2. Procédé de synthèse selon la revendication 1, **caractérisé en ce que** le composé de formule (IX) est de configuration (*S*).

3. Procédé de synthèse selon la revendication 2, **caractérisé en ce que** R représente un atome d'hydrogène et que le produit de la réaction du composé de formule (X) avec l'agent donneur d'hydrure est le composé de formule (XI), cas particulier des composés de formule (VII) : 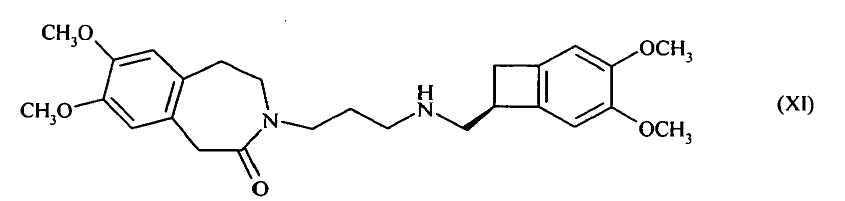 qui peut être *N*-méthylé pour conduire à l'ivabradine de formule (I) : 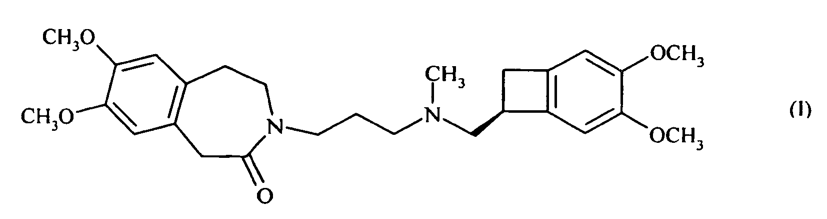 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

4. Procédé de synthèse selon la revendication 2, **caractérisé en ce que** R représente un groupement méthyle et que le produit de la réaction du composé de formule (X) avec l'agent donneur d'hydrure est alors l'ivabradine de formule (I), cas particulier des composés de formule (VII) : 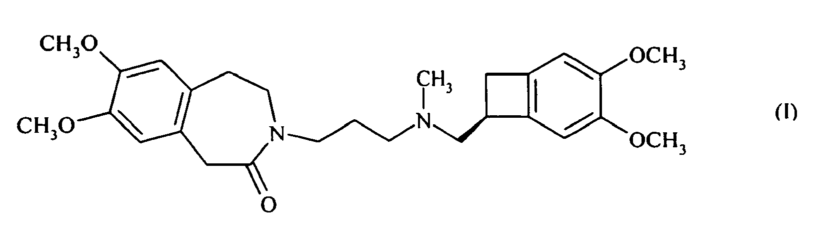 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

5. Procédé de synthèse selon la revendication 1, **caractérisé en ce que** le composé de formule (IX) est sous forme racémique.

6. Procédé de synthèse selon la revendication 5, **caractérisé en ce que** R représente un atome d'hydrogène et que le produit de la réaction du composé de formule (X) avec l'agent donneur d'hydrure est le composé racémique de formule (XII), cas particulier des composés de formule (VII) : 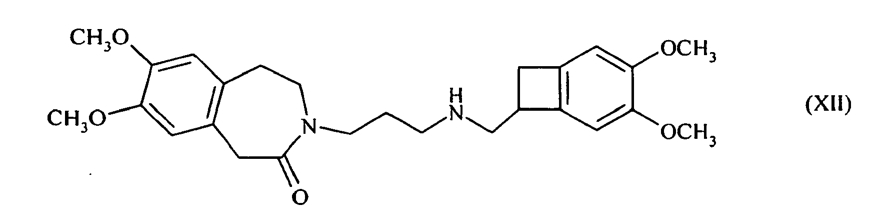 qui peut être *N*-méthylé pour conduire au composé racémique de formule (XIII) : 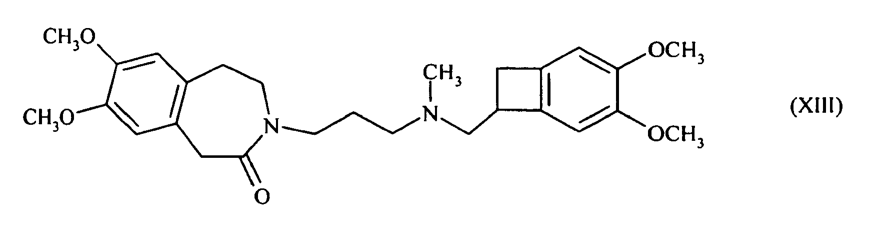 dont la résolution optique conduit à l'ivabradine de formule (I) : 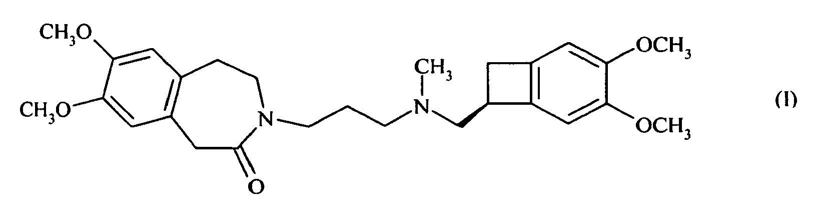 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

7. Procédé de synthèse selon la revendication 5, **caractérisé en ce que** R représente une groupement méthyle et que le produit de la réaction du composé de formule (X) avec l'agent donneur d'hydrure est le composé racémique de formule (XIII), cas particulier des composés de formule (VII) : 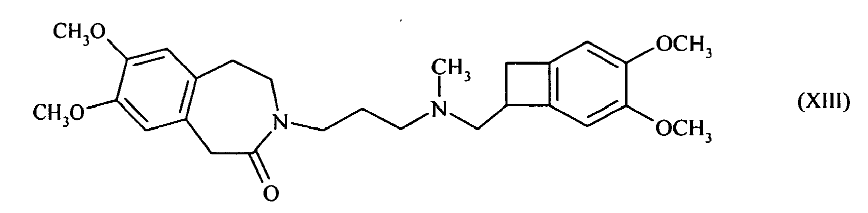 dont la résolution optique conduit à l'ivabradine de formule (I) : 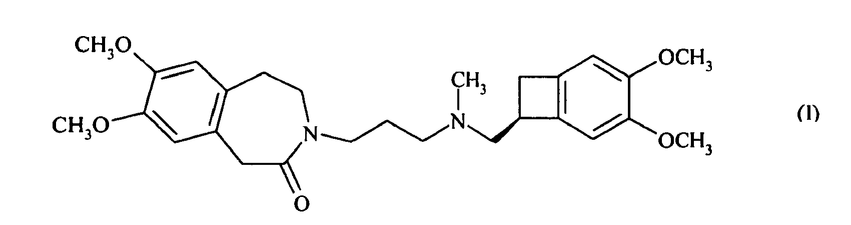 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

8. Procédé de synthèse selon l'une quelconque des revendications 1 à 7, **caractérisé en ce que** le sel de métal de transition ou de lanthanide utilisé pour effectuer la réaction entre le composé de formule (VIII) et le composé de formule (IX) est choisi parmi le chlorure de cuivre(I), le bromure de cuivre(I), l'iodure de cuivre(I), le trifluorométhanesulfonate d'yttrium(III), le trifluorométhanesulfonate de lanthane(III), le trifluorométhanesulfonate de praséodyme(III), le trifluorométhanesulfonate de néodyme(III), le trifluorométhanesulfonate de samarium(III), le trifluorométhanesulfonate d'europium(III), le trifluorométhanesulfonate de gadolinium(III), le trifluorométhanesulfonate de terbium(III), le trifluorométhanesulfonate de dysprosium(III), le trifluorométhanesulfonate d'holmium(III), le trifluorométhanesulfonate d'erbium(III) et le trifluorométhanesulfonate de lutécium(III).

9. Procédé de synthèse selon l'une quelconque des revendications 1 à 8, **caractérisé en ce que** le solvant utilisé pour effectuer la réaction entre le composé de formule (VIII) et le composé de formule (IX) est choisi parmi les solvants alcooliques, le diméthylsulfoxyde, le *N*,*N*-diméthylformamide et la *N*-méthylpyrrolidone.

10. Procédé de synthèse selon l'une quelconque des revendications 1 à 9, **caractérisé en ce que** l'agent donneur d'hydrure utilisé pour effectuer la transformation du composé de formule (X) en composé de formule (VII) est choisi parmi le tétraborohydrure de sodium, le cyanoborohydrure de sodium, le complexe borane-morpholine et le complexe borane-diméthylamine.

11. Composé de formule (VIII) :

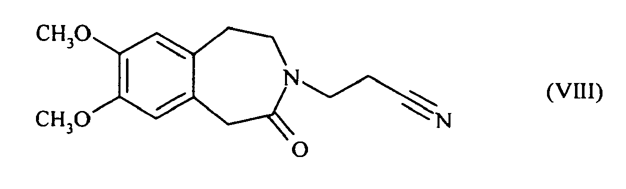

12. Composé de formule (X), sous forme racémique ou optiquement active : 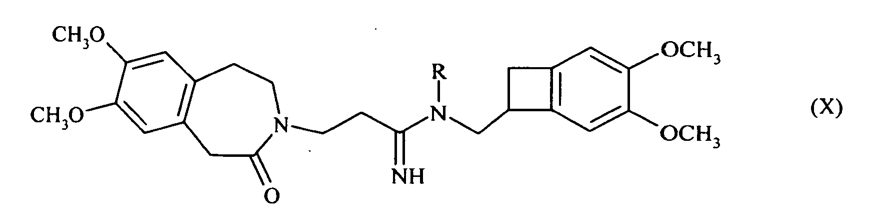 dans laquelle R est tel que défini dans la revendication 1.

## Claims

1. Process for the synthesis of the compound of formula (VII), in racemic or optically active form: 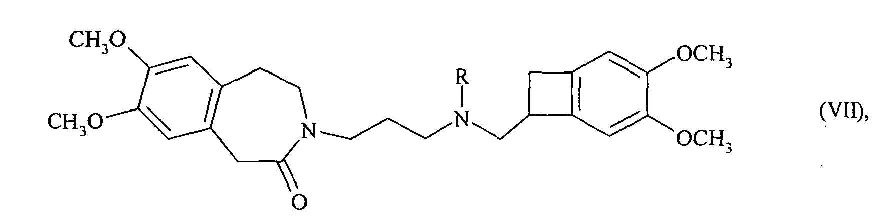 wherein R represents a hydrogen atom or a methyl group,
**characterised in that** the compound of formula (VIII): 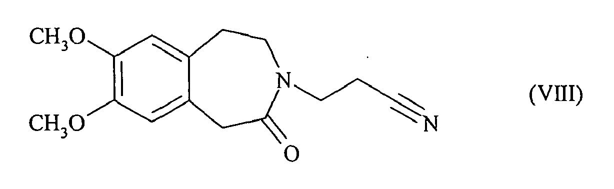 is reacted with the compound of formula (IX), in racemic or optically active form, in the form of the free base or a salt: 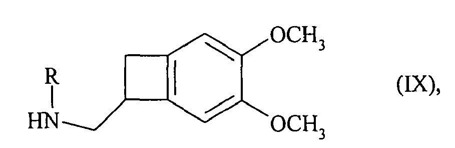 wherein R is as defined hereinbefore,
in the presence of a salt of a transition metal or of a lanthanide,
in a solvent,
to yield the compound of formula (X), in racemic or optically active form: 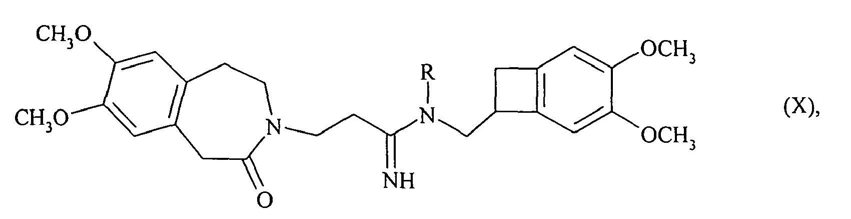 which is converted into the compound of formula (VII) by the action of a hydride donor agent.

2. Synthesis process according to claim 1, **characterised in that** the compound of formula (IX) is of configuration (*S*).

3. Synthesis process according to claim 2, **characterised in that** R represents a hydrogen atom, and **in that** the product of the reaction of the compound of formula (X) with the hydride donor agent is the compound of formula (XI), a particular case of the compounds of formula (VII): 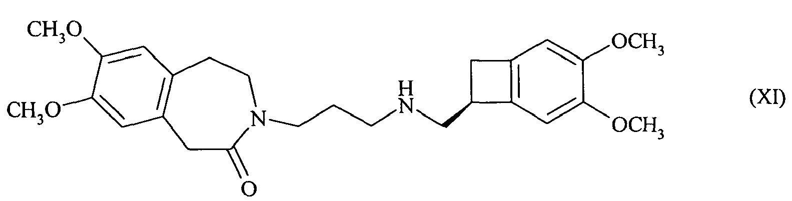 which may be *N*-methylated to yield ivabradine of formula (I): 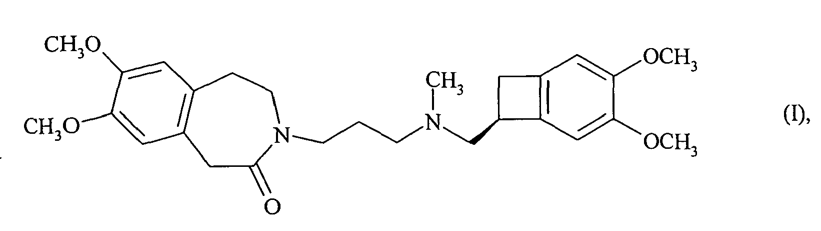 which may optionally be converted into addition salts thereof with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into hydrates thereof.

4. Synthesis process according to claim 2, **characterised in that** R represents a methyl group, and **in that** the product of the reaction of the compound of formula (X) with the hydride donor agent is then ivabradine of formula (I), a particular case of the compounds of formula (VII): 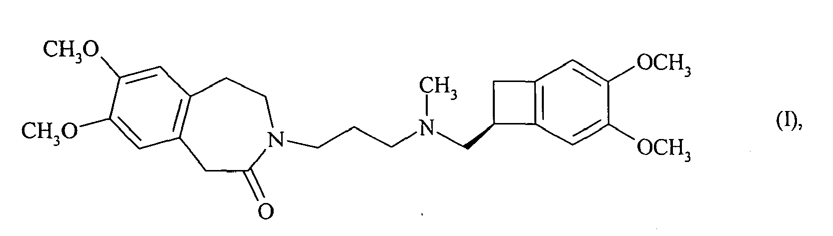 which may optionally be converted into addition salts thereof with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into hydrates thereof.

5. Synthesis process according to claim 1, **characterised in that** the compound of formula (IX) is in racemic form.

6. Synthesis process according to claim 5, **characterised in that** R represents a hydrogen atom, and **in that** the product of the reaction of the compound of formula (X) with the hydride donor agent is the racemic compound of formula (XII), a particular case of the compounds of formula (VII): 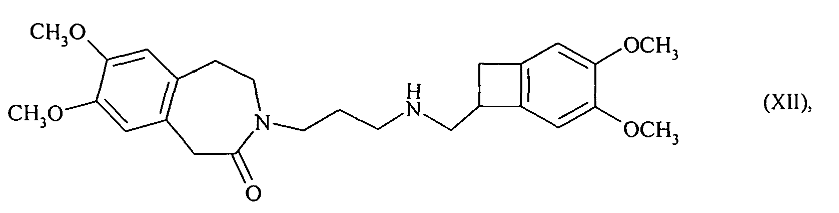 which may be *N*-methylated to yield the racemic compound of formula (XIII): 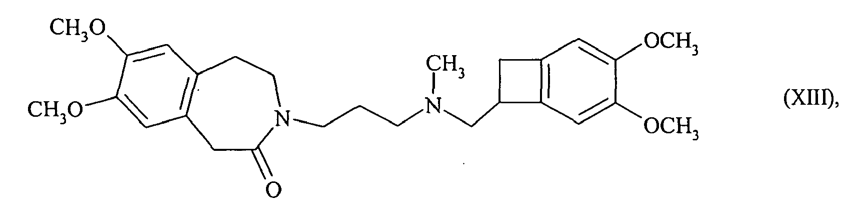 the optical resolution of which yields ivabradine of formula (I): 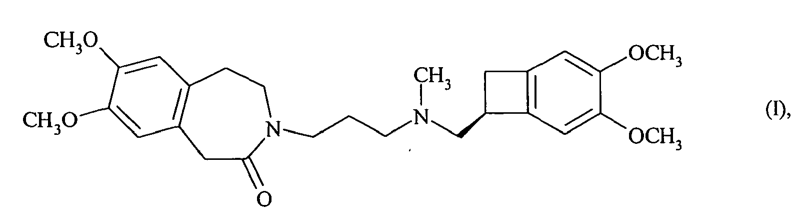 which may optionally be converted into addition salts thereof with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into hydrates thereof.

7. Synthesis process according to claim 5, **characterised in that** R represents a methyl group, and **in that** the product of the reaction of the compound of formula (X) with the hydride donor agent is the racemic compound of formula (XIII), a particular case of the compounds of formula (VII): 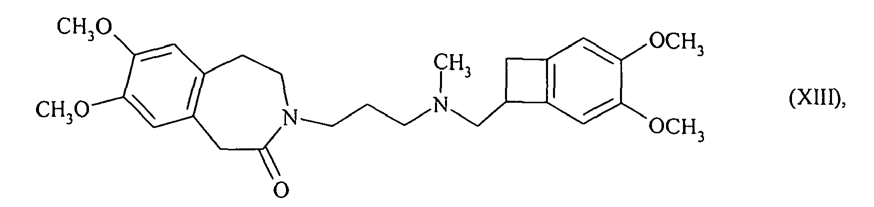 the optical resolution of which yields ivabradine of formula (I): 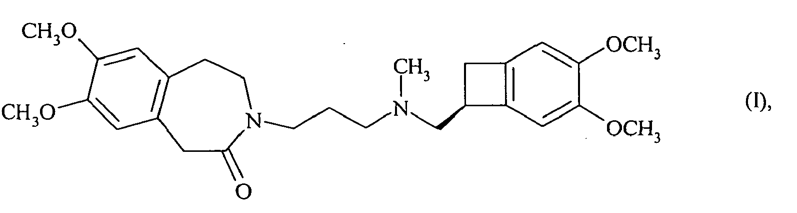 which may optionally be converted into addition salts thereof with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into hydrates thereof.

8. Synthesis process according to any one of claims 1 to 7, **characterised in that** the salt of a transition metal or of a lanthanide used to carry out the reaction between the compound of formula (VIII) and the compound of formula (IX) is selected from copper(I) chloride, copper(I) bromide, copper(I) iodide, yttrium(III) trifluoromethanesulphonate, lanthanum(III) trifluoromethanesulphonate, praseodymium(III) trifluoromethanesulphonate, neodymium(III) trifluoromethanesulphonate, samarium(III) trifluoromethanesulphonate, europium(III) trifluoromethanesulphonate, gadolinium(III) trifluoromethanesulphonate, terbium(III) trifluoromethanesulphonate, dysprosium(III) trifluoromethanesulphonate, holmium(III) trifluoromethanesulphonate, erbium(III) trifluoromethanesulphonate and lutetium(III) trifluoromethanesulphonate.

9. Synthesis process according to any one of claims 1 to 8, **characterised in that** the solvent used to carry out the reaction between the compound of formula (VIII) and the compound of formula (IX) is selected from alcoholic solvents, dimethyl sulphoxide, *N,N*-dimethylformamide and *N*-methylpyrrolidone.

10. Synthesis process according to any one of claims 1 to 9, **characterised in that** the hydride donor agent used to carry out the conversion of the compound of formula (X) to the compound of formula (VII) is selected from sodium tetraborohydride, sodium cyanoborohydride, the complex borane-morpholine and the complex boranedimethylamine.

11. Compound of formula (VIII):

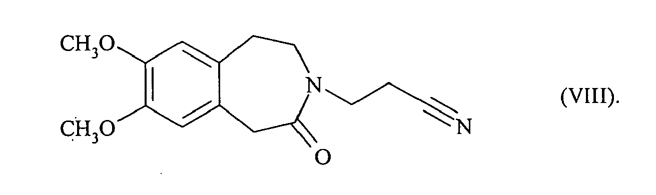

12. Compound of formula (X), in racemic or optically active form: 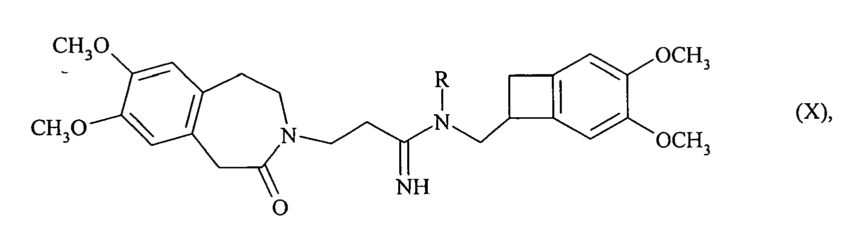 wherein R is as defined in claim 1.

## Patentansprüche

1. Verfahren zur Synthese der Verbindung der Formel (VII) in racemischer oder optisch aktiver Form: 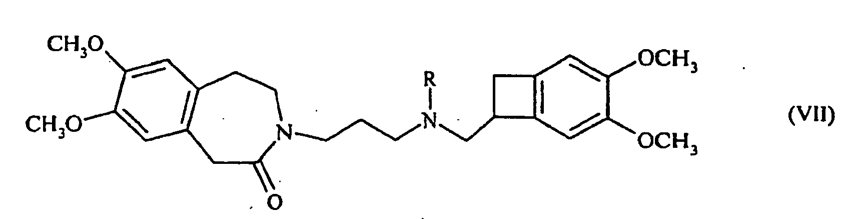 in der R ein Wasserstoffatom oder eine Methylgruppe bedeutet,
**dadurch gekennzeichnet, dass** man die Verbindung der Formel (VIII): 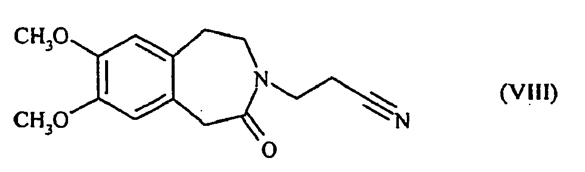 in Gegenwart eines Übergangsmetallsalzes oder eines Lanthanidensalzes in einem Lösungsmittel mit der Verbindung der Formel (IX) in racemischer oder optisch aktiver Form, in Form der freien Base oder des Salzes umsetzt: 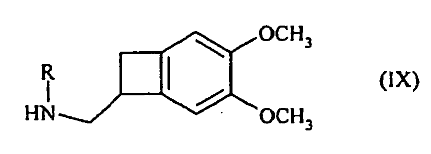 in der R die oben angegebenen Bedeutungen besitzt,
zur Bildung der Verbindung der Formel (X) in racemischer oder optisch aktiver Form: 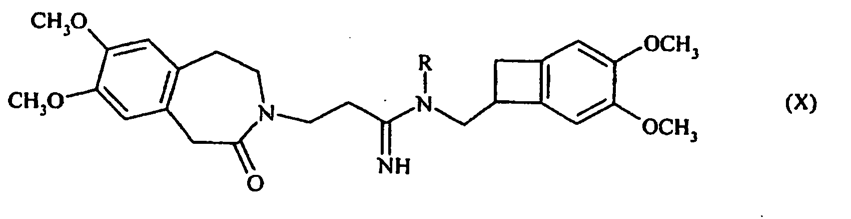 welche durch Einwirkung eines Hydriddonors in die Verbindung der Formel (VII) umgewandelt wird.

2. Syntheseverfahren nach Anspruch 1, **dadurch gekennzeichnet, dass** die Verbindung der Formel (IX) in der Konfiguration (*S*) eingesetzt wird.

3. Syntheseverfahren nach Anspruch 2, **dadurch gekennzeichnet, dass** R ein Wasserstoffatom bedeutet und das Produkt der Reaktion der Verbindung der Formel (X) mit dem Hydriddonor die Verbindung der Formel (XI) ist, ein Sonderfall der Verbindungen der Formel (VII): 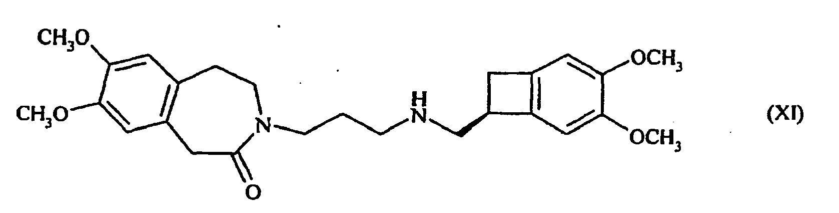 welche *N*-methyliert werden kann zur Bildung von Ivabradin der Formel (I): 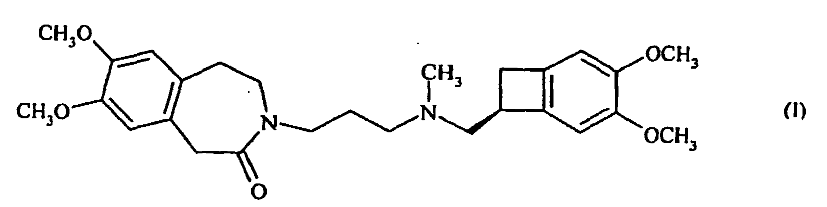 welches gegebenenfalls in seine Additionssalze mit einer pharmazeutisch annehmbaren Säure ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure, und ihre Hydrate umgewandelt werden kann.

4. Syntheseverfahren nach Anspruch 2, **dadurch gekennzeichnet, dass** R eine Methylgruppe bedeutet und das Produkt der Reaktion der Verbindung der Formel (X) mit dem Hydriddonor Ivabradin der Formel (I) ist, ein Sonderfall der Verbindungen der Formel (VII): 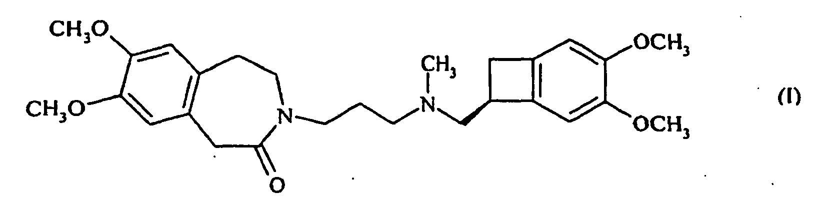 welches gegebenenfalls in seine Additionssalze mit einer pharmazeutisch annehmbaren Säure, ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure, und ihre Hydrate umgewandelt werden kann.

5. Syntheseverfahren nach Anspruch 1, **dadurch gekennzeichnet, dass** die Verbindung der Formel (IX) in racemischer Form eingesetzt wird.

6. Syntheseverfahren nach Anspruch 5, **dadurch gekennzeichnet, dass** R ein Wasserstoffatom bedeutet und das Produkt der Umsetzung der Verbindung der Formel (X) mit dem Hydriddonor die racemische Verbindung der Formel (XII) ist, ein Sonderfall der Verbindungen der Formel (VII): 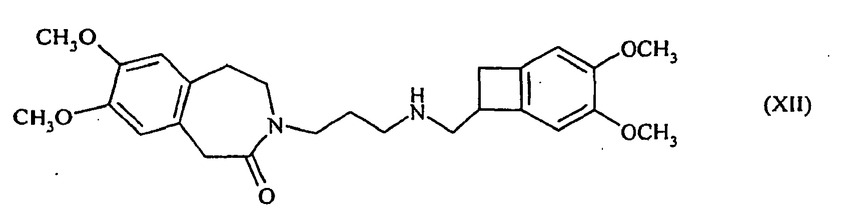 welche *N*-methyliert werden kann zur Bildung der racemischen Verbindung der Formel (XIII): 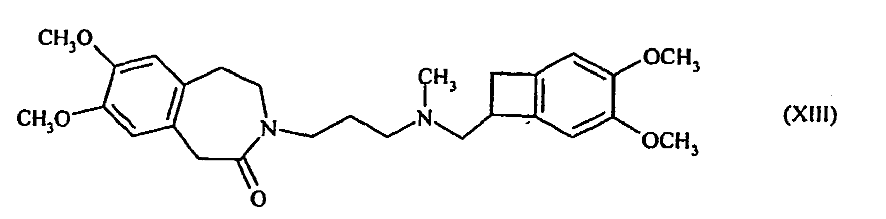 deren optische Trennung zu Ivabradin der Formel (I) führt: 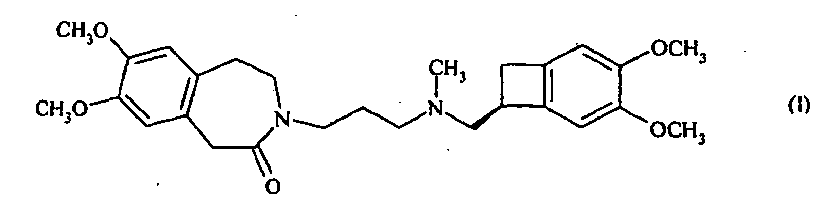 welches gegebenenfalls in seine Additionssalze mit einer pharmazeutisch annehmbaren Säure, ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure, und ihre Hydrate umgewandelt werden kann.

7. Syntheseverfahren nach Anspruch 5, **dadurch gekennzeichnet, dass** R eine Methylgruppe bedeutet und das Produkt der Umsetzung der Verbindung der Formel (X) mit dem Hydriddonor die racemische Verbindung der Formel (XIII) ist, ein Sonderfall der Verbindungen der Formel (VII): 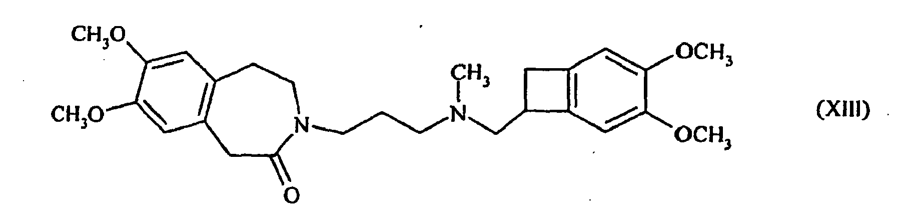 deren optische Trennung zu Ivabradin der Formel (I) führt: 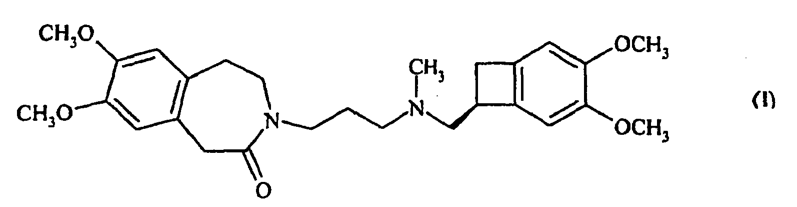 welches gegebenenfalls in seine Additionssalze mit einer pharmazeutisch annehmbaren Säure, ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure, und ihre Hydrate umgewandelt werden kann.

8. Syntheseverfahren nach einem der Ansprüche 1 bis 7, **dadurch gekennzeichnet, dass** das zur Durchführung der Umsetzung der Verbindung der Formel (VIII) mit der Verbindung der Formel (IX) verwendete Übergangsmetallsalz oder Lanthanidensalz ausgewählt wird aus Kupfer(1)-chlorid, Kupfer(I)-bromid, Kupfer(I)-iodid, Yttrium(III)-trifluormethansulfonat, Lanthan(III)-trifluormethansulfonat, Praseodym(III)-trifluormethansulfonat, Neodym(III)-trifluormethansulfonat, Samarium(III)-trifluormethansulfonat, Europium(III)-trifluormethansulfonat, Gadolinium(III)-trifluormethansulfonat, Terbium(III)-triflormethansulfonat, Dysprosium(III)-trifluormethansulfonat, Holmium(III)-trifluormethansulfonat, Erbium(III)-trifluormethansulfonat und Lutetium(III)-trifluormethansulfonat.

9. Syntheseverfahren nach einem der Ansprüche 1 bis 8, **dadurch gekennzeichnet, dass** das für die Durchführung der Umsetzung der Verbindung der Formel (VIII) mit der Verbindung der Formel (IX) verwendete Lösungsmittel ausgewählt wird aus alkoholischen Lösungsmitteln, Dimethylsulfoxid, *N,N-*Dimethylformamid und *N*-Methylpyrrolidon.

10. Syntheseverfahren nach einem der Ansprüche 1 bis 9, **dadurch gekennzeichnet, dass** der für die Umwandlung der Verbindung der Formel (X) in die Verbindung der Formel (VII) verwendete Hydriddonor ausgewählt wird aus Natriumtetraborhydrid, Natriumcyanborhydrid, dem Boran-Morpholin-Komplex und dem Boran-Dimethylamin-Komplex.

11. Verbindung der Formel (VIII):

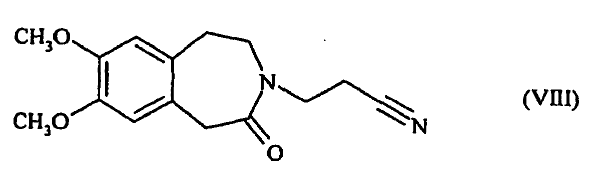

12. Verbindung der Formel (X) in racemischer oder optisch aktiver Form: 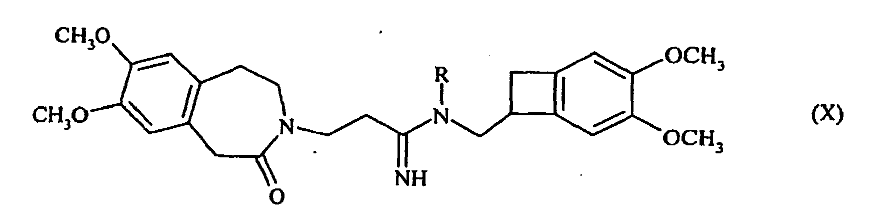 in der R die in Anspruch 1 angegebenen Bedeutungen besitzt.
